# EUROPEAN PATENT SPECIFICATION

(11) **EP 1 439 191 B1**
(45) Date of publication and mention of the grant of the patent: **05.04.2006**
(21) Application number: 03100064.9
(22) Date of filing: 15.01.2003
(51) Int. Cl.: C07K 14/52, C12N 15/19

(54) **Process for the purification of bacterially expressed proteins**
Verfahren zur Reinigung von in Bakterien exprimierten Proteinen
Procédé de purification des protéines produites par la voie bactérienne

(43) Date of publication of application: 21.07.2004
(73) Proprietor: ARES TRADING S.A., 1170 Aubonne (CH)
(72) Inventor: Capponi, Luciano, 00050 Fiumicino (IT); Rossi, Mara, 00179 Roma (IT)
(74) Representative: Serono International S.A.

(56) References cited:
- WO-A-02/28419
- WO-A-98/14467
- MUSACCHIO A ET AL.: "Recombinant Opc meningococcal protein, folded in vitro, elicits bactericidal antibodies after immunization" VACCINE, vol. 15, no. 6/7, 1996, pages 751-758, XP004064539

## Description

### FIELD OF THE INVENTION

This invention relates to a process for the purification of proteins expressed in prokaryotic cells

### BACKGROUND OF THE INVENTION

Recombinant DNA technology makes it possible to produce large amounts of desired proteins. Bacteria are a particularly convenient source for recombinant protein production for purification purposes. They can be grown in very large quantities under well-defined conditions, and they are relatively easy to break open for extraction. Most importantly, molecular cloning techniques allow high levels of expression in bacteria of almost any protein from any organism, bacterial or otherwise. Unfortunately, bacterially expressed proteins are often difficult to purify because of their tendency to precipitate within the cell. The precipitated protein forms inclusion bodies: dense, granular structures that are distributed throughout the cytoplasm. Inclusion body formation is especially common for nonbacterial proteins, although even native bacterial proteins show a tendency to aggregate when they are expressed at very high levels. It is not known exactly how they are formed, but it is thought that the protein is partially or incorrectly folded. The major disadvantage of inclusion bodies is that extraction of the protein of interest generally requires the use of denaturing agents. This can cause problems when a native folded protein is required, because refolding methods are not always 100 percent effective and can be difficult to scale-up.

The advantage of inclusion bodies is that they generally allow greater levels of expression, and they can be separated easily from a large proportion of bacterial cytoplasmic proteins by centrifugation, providing an effective purification step.

For bacterial E. coli lysis, techniques such as mechanical lysis, sonication, enzymatic lysis and detergent lysis are available. These techniques, however, either require special instrumentation or have certain limitations. Most importantly, these methods can neither fully recover soluble recombinant proteins, nor regain inclusion bodies. Thus, the yield of recombinant proteins can be very low. It is necessary to explore alternative methods in order to improve protein yields by recovering both the soluble fraction and insoluble fraction of the recombinant protein.

Musacchio et al. (1996, Vaccine 15:751-758) describe a process for the recovery of the Opc protein expressed in E. coli as inclusion bodies, wherein the recombinant protein is solubilized and purified on a reverse phase column. The eluted protein is finally refolded.

WO 98/14467 discloses a process for the purification of chemokine proteins from inclusion bodies, wherein the protein is purified using reverse phase chromatography after renaturation.

### DESCRIPTION OF THE INVENTION

According to the present invention, a Reverse Phase Chromatography (RPC) step is interposed between the extraction/denaturation step and the subsequent refolding step, resulting in an increased yield of the chemokine and other advantages that will become apparent in the following description. It is believed that the removal of most cell-derived impurities in the RPC step helps in obtaining a higher yield in the subsequent refolding step, although the invention should be considered independently from this hypothesis. Compounds stick to reverse phase HPLC columns in high aqueous mobile phase and are eluted from RP HPLC columns with high organic mobile phase. In RP HPLC compounds are separated based on their hydrophobic character. Since columns are tubular, column dimensions usually take the following format, internal diameter X length (for example 4.6mm X 250mm).

The stationary phase is generally made up of hydrophobic alkyl chains (-CH₂-CH₂-CH₂-CH₃) that interact with the analyte.

In fact, media for RPC are typically highly substituted with hydrophobic ligands and the binding of substances to RPC media is usually very strong and requires organic solvents for elution.

SOURCE™ (Amersham) RPC can be used at full pH range. SOURCE™ RPC are designed for fast, high performance preparative separations of bio -molecules such as proteins, peptides and oligonucleotides. The media have matrices based on rigid, polystyrene/divinyl benzene, with monosized beads of diameters 15 µm or 30 µm respectively.

Pore size distribution is controlled and reproducible. The wide pH stability and high capacity make SOURCE™ RPC media an interesting alternative to silica -based media. The high chemical stability of the matrix offers unmatched flexibility in choice of running and cleaning conditions.

The reverse phase solvents are by convention installed on the HPLC channels A and B. The A solvent by convention is the aqueous solvent (water) and the B solvent by convention is the organic solvent (acetonitrile, methanol, propanol). It is important to follow this convention since the terms A and B are commonly used to refer to the aqueous and organic solvents respectively. The A solvent is generally HPLC grade water with 0.1% acid. The B solvent is generally an HPLC grade organic solvent such as acetonitrile or methanol with 0.1% acid. The acid is used to the improve the chromatographic peak shape and to provide a source of protons i n reverse phase LC/MS. The acids most commonly used are formic acid, triflouroacetic acid, and acetic acid.

As already said,the process of the invention represents in an improvement in this process consisting in that a Reverse Phase Chromatography (RPC) step is interposed between the extraction/denaturation step and the subsequent refolding step. However, all the steps normally carried out in a classical process for purifying a chemokine recombinantly produced in prokaryotic host cells are reported here below just for completeness.

Normally a classical process for purifying a protein produced in prokaryotic cells also includes the following steps:
- Preparation of cell lysates from the host cells
- Isolation of inclusion bodies C
- Solubilization of the aggregated chemokine proteins in the inclusion bodies/denaturation step
- Refolding/renaturation of the solubilized proteins.

### Preparation of cell lysates from the host cells

The chemokine expressed in prokaryotic cells needs first to be extracted from the host cells in which it has been expressed. A variety of methods are available to lyse cells. Which of these methods should be used in a specific case depends on the type of host cells and on the quantity of cells to be lysed.

The first choice to make is that of the nature and the pH of the buffer system we want to use. This depends on:
- the stability of the target protein with respect to pH and the buffering compound.
- the purification procedure.

To avoid time and protein loss caused by an additional buffer exchange step, it is advisable to choose a buffer that is compatible with the first chromatography step (see chromatography). Buffers and their pH ranges are listed in Table 1. The most used buffers are phosphate, Tris - HCl and HEPES NaOH. They are normally used at concentrations of 20-50 mM.

**Table1**

| **Buffer** | **pH range** |
|---|---|
| Citric acid - NaOH | 2.2 - 6.5 |
| Sodium citrate - citric acid | 3.0 - 6.2 |
| Sodium acetate - acetic acid | 3.6 - 5.6 |
| Cacodylic acid sodium salt - HCl | 5.0 - 7.4 |
| MES - NaOH | 5.6 - 6.8 |
| Sodium dihydrogen phosphate disodium hydrogen phosphate | 5.8 - 8.0 |
| Imidazole - HCl | 6.2 - 7.8 |
| MOPS - KOH | 6.6 - 7.8 |
| Triethanolamine hydrochloride - NaOH | 6.8 - 8.8 |
| Tris - HCl | 7.0 - 9.0 |
| HEPES - NaOH | 7.2 - 8.2 |
| Tricine - NaOH | 7.6 - 8.6 |
| Sodium tetraborate - boric acid | 7.6 - 9.2 |
| Bicine - NaOH | 7.7 - 8.9 |
| Glycine - NaOH | 8.6 - 10.6 |

Depending on the target chemokine protein, it may be necessary to add compounds to the lysis buffer to improve the stability of the target protein and to keep the protein in solution. The most used additives, their effective concentrations, an d their general purpose are listed in Table 2.

**Table 2**

| **Class of Additive** | **Example** | **Concentration** | **Purpose** |
|---|---|---|---|
| Salts | NaCl, KCl, (NH₄)₂SO₄ | 50-150 mM | maintain ionic strength of medium |
| Detergents | Deoxycholate, Triton X-100 | 0.1-1% | solubilization of poorly soluble proteins |
| Glycerol | | 5-10% | stabilization |
| Glucose or sucrose | | 25 mM | Stabilize lysosymal membranes, reduce protease release |
| Metal chelators | EDTA, EGTA | 1 mM | reduce oxidation damage, chelate metal ions |
| Reducing agents | 2-Mercaptoethanol, DTT | 0.05% | reduce oxidation |
| Ligands, metal ions | Mg²⁺, ATP, GTP | 1-10 mM | stabilization |

An important class of additives is the protease inhibitors. ln general cell disruption leads to the release of proteolytic enzymes, which could lower the overall yield. To control this undesirable proteolysis it may be necessary to add a cocktail of protease inhibitors to the cell suspension. Since many of these compounds are not very stable in aqueous solutions it is important that they are added to the lysis buffer from a stock solution in an organic solvent (methanol, ethanol, isopropanol, or DMSO) immediately before use.

Although it is impossible to give a general lysis buffer, a good starting buffer would be:
50 mM Tris-HCl pH 7.5
100 mM NaCl
1 mM DTT (Dithiothreitol)
5% glycerol (possibly).

Most lysis methods cause the release of nucleic acids (DNA and RNA). These have to be removed because they can cause viscosity problems or due to their interference with subsequent chromatographic steps. Different methods exists:
- Enzymatic digestion by the addition of DNase I (1 µg/ml) to the cell lysate. The mixture is incubated on ice for 10-15 min.
- Mechanical breakdown by shearing during sonication. When the French Pressure Cell is used it is advisable to add DNase to the cell suspension.
- Precipitation by treatment with polyethyleneimine (0.1 °to (w/v)) or protamine sulphate (1% (w/v)) followed by centrifugation. Add the precipitants to the cell lysate and incubate the solution for 30 min at 4°C.

Different methods are used for the preparation of cell lysates from *E. coli* cells.

Sonication is the most popular technique for lysing small quantities of cells (1-6 L of cell culture). Cells are lysed by liquid shear and cavitation. DNA is also sheared during sonication, so it is not necessary to add DNase to the cell suspension. The main problem is controlling the temperature. This is addressed by keeping the suspension on ice and using a number of short pulses (5-10 sec) with pauses (10-30 sec) to reestablish a low temperature. For cell quantities larg er than 50 g the method is of limited value because of the difficulty in maintaining low temperatures and the long sonication times needed to reach adequate lysis.

Homogenizers are the most common devices to lyse bacteria. The presses lyse cells by pressurizing the cell suspension and suddenly releasing the pressure. This creates a liquid shear capable of lysing cells. Typical operating pressures for the older type of homogenizers, the French press and Manton-Gaulin homogenizer, are 6000-10,000 psi. Multiple (2-3) passes are generally required to achieve adequate lysis. The high operating pressures, however, result in a rise in operating temperatures.

Therefore, pressure cells are cooled (4°C) prior to use. In addition to temperature control, care should be taken to avoid inactivating proteins by foaming.

Modem homogenizers are often continuous and can be operated at higher pressures. At EMBL we have an Avestin Emulsiflex-C5 that efficiently lyzes *E. coli* cells in one passage at 15,000 psi (100 MPa).

Enzymatic lysis is based on the digestion of the peptidoglycan layer of the bacterial cell wall by lysosyme. Gram-negative bacteria, however, have an outer membrane that is external to the cell wall and needs to be permeabilized to expose the peptidoglycan layer. Tris, often used as a buffer in lysis methods, effectively permeabilizes outer membranes. This effect can be enhanced by the addition of EDTA (1 mM). EDTA chelates the magnesium ions that stabilize membranes. During cell lysis often a lot of DNA is liberated and it becomes necessary to add DNase (1 mg/ml) to reduce the viscosity of the preparation. Enzymatic cell lysis can be carried out on any scale but for large-scale preparations the lysosyme and DNase can get expensive. To increase the level of cell lysis the solution can be sonicated (see above).

An alternative lysis method is to freeze the cells directly in liquid nitrogen and ground the frozen cells to a powder using a mortar and pestle that are chilled with liquid nitrogen. The powder can be stored indefinitely at -80°C and the cell lysate can be prepared by adding the powder to 5 volumes of buffer.

The extent of the solubilization and the stability of the solubilized protein depend on the detergent type and concentration. It is not possible to give general rules for either of these variables and they have to be optimized experimentally. Important for the solubilization is the detergent-to-protein ratio. At low ratios (1:10) the membranes are lysed and large complexes of are formed containing protein, detergent, and membrane lipids. With progressively larger ratios smaller complexes are obtained. Finally, at ratios of 10:1 to 20:1 individual detergent-protein complexes are formed free of membrane lipids. To determine the optimal conditions it is important to vary both the detergent and the protein concentration.

Commonly used detergents and their critical micel concentrations (cmc) are listed in Table 3.

**Table 3**

| **Class of detergent** | **examples** | **cmc (mM)** |
|---|---|---|
| Non-ionic | Triton X-100 | 0.30 |
| | octylglucoside | 25 |
| Zwitterionic | CHAPS | 6.5 |
| | Zwittergent 3-12 | 3.6 |
| Ionic | sodium deoxycholate | 4 (at pH>8) |

### Isolation of inclusion bodies

In the cell there is competition between folding and aggregations. In many cases and in several host systems, recombinant proteins accumulate intracellularly in insoluble aggregates. The proteins in these so-called inclusion bodies are mostly inactive and denatured. In addition, dimers and multimers may be present. However, the expression of recombinant proteins in inclusion bodies can also be advantageous:
- the recombinant protein deposited in inclusion bodies can be 50% or more of the total cellular protein.
- the inclusion bodies often contain almost exclusively the overexpressed protein.
- in inclusion bodies the protein is protected from proteolytic degradation.
- expression in inclusion bodies will protect the cell against the toxicity of the recombinant protein.
The major problem is to recover biologically active and/or soluble protein in high yield. In order to accomplish this, the protei n in the inclusion bodies must by solubilized and refolded *in vitro.* This procedure is carried out in three phases:

Inclusion bodies have a relatively high density and, therefore, can be pelleted by centrifugation. Cells are usually disrupted by high-pressure homogenization (optionally following a lysosyme treatment). It is important that cell lysis is complete, because intact cells sediment together with the inclusion bodies, thus contaminating the preparation. After centrifugation, the pellet is washed with buffer containing either low concentrations of chaotropic agents (*e.g*. 0.5 -1 M guanidine-HCl or urea) or detergents (e.g. 1% Triton X-100 or 1 mg/ml sodium deoxycholate). This wash step is necessary to remove contaminants, especially proteins (proteases), that may have absorbed onto the hydrophobic inclusion bodies during processing.

### Solubilization of the aggregated proteins in the inclusion bodies

The washed inclusion bodies are resuspended and incubated in buffer containing a strong denaturant and a reducing agent (usually 20 mM DTT or b-mercaptoethanol). The addition of a reducing agent keeps all cysteines in the reduced state and cleaves disulfide bonds formed during the preparation. Incubation temperatures above 30°C are typically used to facilitate the solubilization process.

Optimal conditions for solubilization are protein specific and have to be determined for each protein.

After solubilization the solution can be centrifuged or filtered to remove remaining aggregates which could act as nuclei to trigger aggregation during refolding.

According to a specific embodiment of the invention reported here after in the Examples, this step is indicated as the Step 1 of the specific process described.

### Refolding of the solubilized proteins

Refolding of the solubilized proteins is initiated by the removal of the denaturant. The efficiency of refolding depends on the competition between correct folding and aggregation. To slow down the aggreagtion process refolding is usually carried out at low protein concentrations, in the range of 10-100 mg/ml. Furthermore, refolding conditions must be optimized for each individual protein. Important variables are the buffer composition (pH, ionic strength) the temperature and the additives (often in combination).

If proteins contain disulfide bonds, the refolding buffer has to be supplemented with a redox system. The addition of a mixture of reduced and oxidized forms (1-3 mM reduced thiol and a 5:1 to 1:1 ratio of reduced to oxidixed thiol) of low molecular weight thiol reagent usually provides the appropriate redox potential to allow formation and reshuffling of disulfide bonds. The most commonly used redox shuffling reagents are reduced and oxidized glutathione, but also cysteine and cysteamine are used.

For certain protein, probably due to low solubility of folding intermediates, this procedure is not very effective. Alternatively, the protein is completely oxidized in the presence of a large excess of oxidized glutathione, followed by dilution in refolding buffer containing catalytic amounts of reduced glutathione.

Different methods for the refolding of proteins have been described. The most used method is the removal of the solubilizing agent by dialysis. During dialysis the concentration of the solubilizing agent decreases slowly which allows the protein to refold optimally. The ratio of the volumes of the sample and the dialysis buffer should be as such that at the equilibrium concentration of the solubilizing agent the protein has completely refolded.

The concentration of the solubilizing agent is decreased by dilution allowing the protein to refold. Usually the dilution is carried out slowly by step -wise addition of buffer or by continuous addition using a pump.

During dialysis and slow dilution the protein is exposed for an extended period of time to an intermediate concentration of the solubilizing agent (2-4 M urea or guanidine-HCl) where it is not yet folded but no longer denatured and thus extremely prone to aggregation. This could be prevented by the rapidly dilution of the solubilized protein solution into the refolding buffer. Aggregation during this process can be limited by adding mild solubilizing agents to the refolding buffer, such as non-detergent sulfobetaines.

In order to keep the concentration of th e unfolded protein low, thus limiting aggregation, aliquots of denatured protein are added at defined time points to the refolding buffer. The time intervals between two pulses have to be optimized for each individual protein. The process is stopped when the concentration of denaturant reaches a critical level with respect to refolding of the specific protein.

According to a specific embodiment of the invention reported here after in the Examples, this step is indicated as step 3 of the specific process des cribed.

The solubilizing agent is removed using a chromatographic step. The application of different chromatography methods have been described:
- size exclusion chromatography (e.g. gel filtration on a Superdex 75 column)
- ion exchange chromatography
- affinity chromatography (e.g. IMAC using Chelating Sepharose or Ni-NTA agarose)

The denaturant is removed while the protein is slow migrating through the column or bound to the matrix. This usually gives a high yield of active protein even at protein concentrations in the mg/ml range.

### EXAMPLES

The invention will now be described in detail with respect to the purification of a Chemokine expressed in E. Coli. Chemokines constitute a family of small pro-inflammatory cytokines with leukocyte chemotactic and activating properties. Depending on the position of the first conserved cysteines, the chemokine family can be divided in C-C, C-X-C and C-X₃-C chemokines (Baggiolini M. et al., Adv Immunol. 1994, 55:97-179; Baggiolini M. et al., Annu Rev lmmunol. 1997,15:675-705; Taub D. et al., Cytokine Growth Factor Rev. 1996,7(4):355-76).

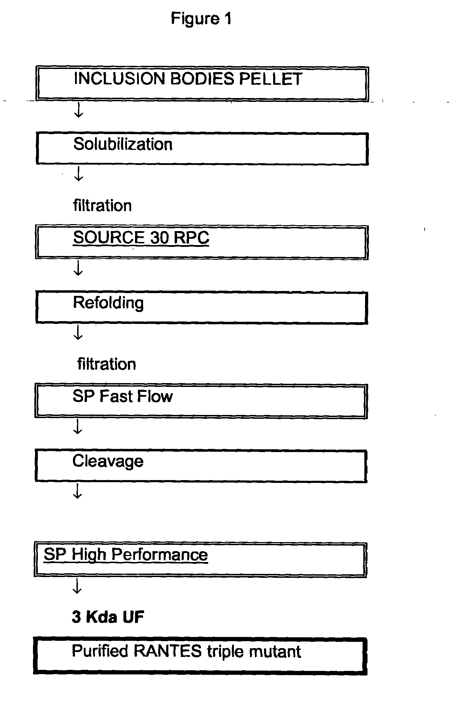

In particular, the detailed description that follows reports the purification of a triple mutant of human RANTES. This protein in the mature form (that is, after cleavage of the leader sequence MKKKWPR), has the sequence of SEQ ID NO: 1 and will be referred to in the further part of this specification as RANTES triple mutant. The overall process is summarized by the flow chart presented in Figure 1, starting from the step of "solubilization of the aggregated proteins in the inclusion bodies". The two previous classical steps of preparation if cell lysates and isolation of inclusion bodies are carried out using the methods known in the art and/or as described before.

### Step 1 - Solubilization of the Aggregated Proteins the Inclusion Bodies

60-90 gr of inclusion bodies containing RANTES triple mutant are thawed. The pellet is dissolved with 450-800 ml of solubilization buffer (6M Guanidinium Chloride, 0.1 M Tris/HCl, 2mM DTT, pH 7.5+/-0.1) by means of the homogeniser Polytron until big particles are not visible anymore (about 5 minutes). Once homogenised, the solution is brought at a temperature of 60 ± 1 °C for 30'.

Then the solution is allowed to reach room temperature and filtered with a 1.2 µm membrane.

The material in usually processed as soon as it is prepared, in alternative it can be stored at -80°C.

### Step 2 - Reverse Phase Chromatography On Source 30 RPC

### Buffers

### Pre-equilibration buffer: 0.1 M TRIS/HCl pH 7.5±0.1, 5% (v/v) Acetonitrile.

12.1 g. of Tris/hydroxymethilaminomethane is added to 800-900 ml of purified water. The pH is adjusted to 7.5 with HCl. 50ml of Acetonitrile is added and the solution is brought to 1 liter with purified water.

### Equilibration buffer: 0.1 M TRIS/HCl pH 7.5±0.1, 6M Guanidine and 2 mM DTT, 5% (v/v) Acetonitrile.

6M guanidine and 2mM DTT are added to 600 ml of pre-equilibration buffer under stirring. After dissolution is completed, the solution is brought to 1 liter with pre - equilibration buffer.

### Washing buffer: 5% (v/v) Acetonitrile. 0.1% TFA in water

50 ml of acetonitrile and a vial of TFA are added to 900 ml of purified water and the solution is brought to 1 liter with purified water.

### Elution buffer: 35% (v/v) Acetonitrile, 0.1 % TFA in water

350 ml of acetonitrile and a vial of TFA are added to 500-600 ml of purified water and the solution is brought to 1 liter with purified water.

### Regeneration buffer: NaOH 0.5 M. 60% n -propanol

20 gr of NaOH is added to 600 ml of n -propanol and the solution is brought up to 1 liter with purified water.

### Storage buffer: NaOH 10 mM

0.4 gr of NaOH is added to 1000 ml of purified water

### Procedure

A column packed with Source 30RPC resin is flushed with at least 1 BV of NaOH 0.5M and subsequently with purified water until the pH reaches neutral values. Then the column is equilibrated with 3 BV of pre-equilibration buffer followed by 2-3BV of the equilibration buffer. The pH is checked and washing is continued if the parameters of the column's effluent are out of target values: pH 7.5±0.1.

The solubilized material prepared as described above is then loaded on the resin in the range of 5-18 mg of RANTES triple mutant/ ml of resin. When sample loading is completed, the column is flushed with 3 -4 BV of equilibration buffer 0.1 M Tris/HCl + 5% Acetonitrile, pH 7.5± 0.1. The flow trough and the wash are collected together.

Then the column is flushed with the washing buffer: 5% Acetonitrile + 0.1 % TFA in water until the pH of the column effluent is acidic (control with the pH indicator).

Elution is started with the elution buffer: 35% Acetonitrile + 0.1% TFA in water. The fractions are collected as soon as the OD signal starts to rise. About 3 -4 BV are collected as elution fraction.

The protein content of the fraction is calculated by th e OD 280 nm analysis (ε=2.1). The fraction is stored at +5±3 °C waiting for the next step of refolding.

After elution is completed flush the column with at least 4 BV of regeneration buffer followed by 3BV of purified water.

The column is flushed with at I east 3 BV of NaOH 0.5M, and then the column is rinsed with purified water.

Afterwards the column is flushed with at least 3 BV of storage buffer and stored at room temperature until the next cycle.

The step here described is very efficient in the removal of cell impurities and gives a solution containing semipurified RANTES triple mutant ready to be submitted to the refolding step.

### Step 3 - Refolding

In this step the unfolded RANTES triple mutant, eluted from the RPC column, is refolded by means of diluti on in a suitable buffer and the use of a redox system.

### Refolding buffer: 0.1 M Tris/HCl pH 7.5, 0.2 mM glutathione reduced form, 0.02 mM Glutathione oxidized form: cond. 6.0±1 mSi/cm

12,1 gr of Tris, 61.5 mg of glutathione reduced and 12.2 mg of glutathio ne oxidized are added to 900 ml of purified water under stirring, bring to pH 7.5±0.1 with concentrated HCl and make up to 1liter. The solution is then stored at refrigerated temperature +5°C ±3°C for not more than two days.

### Procedure

This step is performed at refrigerated temperature (+5±3°C). The RPC elution fraction is diluted by adding it overnight drop by drop under mild stirring into a suitable volume of refolding buffer in order to reach a theoretical final concentration (calculated by OD) of 0.2-0.4 mg/ml.

Step 2 described before is very efficient in the removal of cells impurities giving a solution containing semi-purified RANTES triple mutant ready to be submitted to the refolding step.

### Step 4 - IEC on SP Sepharose FF

### Buffers And Solutions

### Equilibration buffer: 0.1 M Tris/HCl pH 7.5±0.1. conductivity 6.0±1 mS/cm

12.1 g of Tris is added to 900 ml of purified water under stirring, pH is brought to 7.5±0.1 with 37% HCl and the solution is brought up to 1 liter. Conductivity is checked. The solution is stored at room temperature for not more than two days.

### Elution buffer: 0.1 M Tris/HCl pH 7.5±0.1, 0.6 M NaCl, conductivity 57.0±2.0 mS/cm

12.1 g of Tris and 35 g of NaCl are added to 900 ml of purified water under stirring, the pH is brought to 7.5±0.1 with HCl concentrated and the solution is brought up to 1 litre. Conductivity is checked. This solution is stored at room temperature +20°C±5°C and used within two days.

### Regeneration buffer::1.5 M NaCl

87.6 g of NaCl is dissolved in 900 ml of purified water under stirring and the solution is brought up to 1 litre. This solution is stored at room temperature +20°C±5°C and used within two days.

### Sanitizing solution: 0.5M NaOH

20 g of NaOH is dissolved in 900 ml of purified water under stirring and the solution is brought up to 1 litre.

### Storage solution: 0.01 M NaOH

0.4 g of NaOH is dissolved in 900 ml of purified water under stirring and the solution is brought up to 1liter. This solution is stored at room temperature +20±5°C and used within two days.

### Procedure

The column packed with SP Sepharose F resin is flushed with 3BV of NaOH 0.5 M followed by purified water until the pH goes down to neutral values (pH indicator paper). Then the column is flushed again with 5 or more BV of equilibration, buff er. pH and conductivity are checked and the wash is continued, if the parameters of the column's effluent are out of target values: pH 7.5±0.1, conductivity 6±1 mS/cm.

Then the filtered post refolding material is loaded and the unbound fraction is collected.

When sample loading is completed, the column is flushed with 1-2 BV of equilibration buffer and collection is continued. At this point usually the UV signal is on the baseline.

Then the column is flushed with the elution buffer. The elution fraction is started to be collected when the UV signal starts to rise, usually after the first 0.8 BV. 3BV of eluate is collected and this fraction is stored at 2-8°C until the next step of cleavage.

After elution is completed, the column is flushed with at least 4 BV of regeneration buffer.

The column is flushed with at least 3 BV of NaOH 0.5M and then rinsed with 3 BV of purified water. The column is flushed with at least 3 BV of storage solution and stored until the next cycle.

### Step 5 - Cleavage

The cleavage step is used to remove the -RANTES triple mutant leader sequence (MKKKWPR) from the correct sequence.

### Procedure

The cleavage is performed at a temperature of 37 ±1°C in a thermostatic oven.

The quantity of trypsin to be added is calculated on a ratio of 1:10000 in respect of the RANTES triple mutant content calculated by RP-HPLC analysis.

The trypsin is added as soon as the material to be cleaved has reached the target temperature.

The ongoing of the cleavage is followed by the RP-HPLC analysis that is able to separate the not cleaved peak from the cleaved one. Usually the cleavage is completed after about 3 hours. Just after the cleavage is completed, in order to stop the reaction, the pH of the solution is brought to 3.2 with concentrated phosphoric acid.

### Step 6 - IEC on SP Sepharose HP

### Buffers And Solutions

### Buffer A -Equilibration-: 50 mM Ammonium Acetate pH 3.2±0.2, conductivity 0.4 ±0.1 mSi/cm

2.8 ml of acetic acid is added to 900 ml of purified water under stirring, the pH is brought to 3.2±0.2 with ammonia 25% and the solution is brought up to 1 litre. Conductivity is checked. The solution is stored at room temperature 20°C ±5°C for not more than two days.

### Buffer B -Elution-: 50 mM Ammonium Acetate pH 3.2±0.2, 1 M NaCl conductivity 83 ± 2 mS/cm

2.8 ml of acetic acid and 58.4 g of Nacl are added to 900 ml of purified water under stirring, the pH is brought to 3.2±0.2 with ammonia 25% and the solution is brought up to 1 litre. pH and conductivity are checked.

The solution is stored at room temperature 20°C ±5°C for not more than two days.

### Sanitizing solution: 0.5M NaOH

20 g of NaOH is dissolved in 900 ml of purified water under stirring and the solution is brought up to 1 litre.

### Storage solution: 0.01 M NaOH

0.4 g of NaOH is dissolved in 900 ml of purified water under stirring and the solution is brought up to 1 litre. This solution is stored at room temperature +20±5°C and used within five days.

### Procedure

Post cleavage material is diluted 1:2 with purified water in order to obtain a conductivity of the solution around 30 mSi/cm

This chromatographic step requires the use of a system able to perform gradients between the equilibration and elution buffers.

The column is flushed with at least 3BV of NaOH 0.5 M and rinsed with purified water until the pH is neutral. Then the column is flushed with column 4-5 or more BV of equilibration buffer. pH and conductivity are checked and washing is continued, if the parameters of the column's effluent are out of target values:
pH 3.2±0.2 and conductivity 0.4 ± 0.1mS/cm

The starting material prepared as above is loaded on the column. As soon as the loading is completed, the column is flushed with 2 -3 BV of equilibration buffer, until the UV signal reaches the baseline and then the column is flushed with a buffer composed by 60% of equilibration buffer and 40% of elution buffer. About 5-6 BV of this washing fraction is collected.

Elution is performed with a gradient between 40% and 100% of buffer B in 20 BV. The elution is collected in fractions (1 min. each one) that will be pooled later on, following the chromatogram pattern that usually shows the peaks from the peptide sequence, the cleaved (and correct) one and the uncleaved molecule. Based on the chromatogram the fractions that form the peak of interest are collect ed in order to form the elution fraction.

Then the column is flushed with at least 3-4 BV of NaOH and then rinsed with 3-4 BV of purified water.

The column is flushed with at least 3 BV of storage solution and stored until the next cycle.

### Step 7 - Bulk Ultrafiltration

### Buffers And Solutions

### Buffer: 50 mM Ammonium Acetate, pH 4.0±0.1 conductivity 1.0±0.2 mS/cm

2.8 ml of Acetic Acid is added to 900 ml of purified water, under stirring. The solution is brought up to 1 litre. The pH is brought to 4.0 ± 0.1 with 25% ammonia and conductivity is checked. This solution is stored at room temperature +20±5°C and used within 1 day.

### Sanitizing solution: 0.5 M NaOH

20 g of sodium hydroxide are dissolved in 900 ml of purified water under stirring. The solution is brought up to 1 litre and stored at room temperature.

### Storage solution: 0.05 M NaOH

2 g of sodium hydroxide is dissolved in 900 ml of purified water under stirring, the solution is brought up to 1 litre, stored at room temperature +20±5°C and used within three days.

### Procedure

The ultafiltration system is assembled with a regenerated cellulose membrane at cut off 3KD (Millipore).

The ultrafilters are sanitized with 200 ml of NaOH 0.5 M by recycling the alkaline solution for not less than 30 minutes and then rinsed with purified water until permeate pH is below 7.5.

The SP HP pool elution solution is recirculated on the system at a constant pression of slightly less than 0.5 bar and purified water is added, trying to maintain the volume constant. Conductivity is checked and washing is continued until it reaches values below 1 mSi/cm.

Then the column is washed with the bulk buffer: 50 mM Ammonium Acetate pH 4.0± 0.1, cond. 1.0±0.2 mSi/cm until permeate reaches the same values.

The retentate fraction is collected and the system is washed recirculating it with the same buffer until no foam is visible anymore.

The ultrafilters are washed and sanitized by recycling around 200 ml of 0.5 M NaOH for at least 30 minutes, then rinsed ultrafilters with purified water until the permeate pH is below 7.5. The ultrafilters are then stored in 0.05M NaOH at +20 ±5°C until the next cycle.

### Analysis

The preliminary analysis for quantitation of RANTES triple mutant has been set up to measure the concentration of the molecule in the i n process samples to monitor the yield of the process and to monitor both refolding and cleavage steps. Here follows the description of the analyses.3

| **Equipment** | **Analytical HPLC system equipped with column oven** | | |
|---|---|---|---|
| Column | Symmetry C18, 3.5 µ, 4.6x 75mm | | |
| Eluent A | 0.1% aqueous TFA | | |
| Eluent B | 0.1 % TFA in acetonitrile | | |
| Eluent C (for column storage) | 50% acetonitrile in water | | |
| **Method** | | | |
| UV detection | 214 nm | | |
| Temperature | 45°C | | |
| Injection time | 50 minutes | | |
| Injection volume | 10-100 µl | | |

| **Gradient** | Time | %A | %B |
|---|---|---|---|
| | 0 | 75 | 25 |
| | 25' | 65 | 35 |
| | 25.1' | 20 | 80 |
| | 30' | 20 | 80 |
| | *30.1' | 75 | 25 |
| | *38' | 75 | 25 |
| | *38.1' | 75 | 25 |
| **Flow rate** | 1 ml/minute | | |
| | * step performed at 1.2 ml/min | | |
| **Reference sample** | Purified uncleaved RANTES triple mutant at the concentration of 0.65 g/ml by OD (E=21) | | |

The overall recovery of the purification process estimated by RP-HPLC resulted to be over 30%, which is a very good result compared with the data in the literature; the purity by RP-HPLC is >90%, by SE-HPLC is > 95% and the HCP content, analysed with an ELISA commercial kit, is < 100 ppm.

A panel of such other analyses as SDS, IEF and Maldi TOF on the several batches of drug substances produced confirmed the consistency of the process both as purity and quality of the molecule.

## Claims

1. A process for recovering a chemokine expressed in prokaryotic host cells as inclusion bodies and its subsequent purification, **characterized in that** it interposes a Reverse Phase Chromatography step between the step of solubilization of the aggregated proteins in the inclusion bodies/denaturation and the renaturation/refolding step.

2. A process according to claim 1, **characterized in that** the following steps are performed :
• Solubilizing the aggregated chemokine proteins in the inclusion bodies;
• Subjecting the solubilized chemokine proteins to a Reverse Phase Chromatography;
• Subjecting the obtained product to a renaturation /refolding step;
• Subjecting the obtained product to a chromatographic step selected from size exclusion chromatography, ion exchange chromatography, affinity chromatography.

3. A process according to claim 1 or 2, **characterized in that** the following steps are performed:.
• Solubilizing the aggregated chemokine proteins in the inclusion bodies;
• Subjecting the solubilized chemokine protein to a Reverse Phase Chromatography;
• Subjecting the obtained product to a renaturation /refolding step;
• Subjecting the obtained product to two ion exchange chromatography steps.

4. A process according to any of the preceding claims, whereby after the solubilizing and/or refolding step a filtration step is performed.

5. The process according to any of the preceding claims, whereby the prokaryotic cells are bacterial cells.

6. The process according to claim 5, whereby the bacterial cells are E. Coli cells.

7. The process of claim 6, wherein the chemokine is the chemokine mutant of SEQ ID NO: 1.

## Patentansprüche

1. Verfahren zur Gewinnung eines in prokaryotischen Wirtszellen als Einschlußkörper exprimierten Chemokins und seine anschließende Reinigung, **dadurch gekennzeichnet, daß** ein Umkehrphasenchromatographie-Schritt zwischen den Schritt der Solubilisierung der aggregierten Proteine in den Einschlußkörpern/Denaturierung und den Renaturierungs-/Rückfaltungsschritt eingeschoben wird.

2. Verfahren gemäß Anspruch 1, **dadurch gekennzeichnet, daß** die folgenden Schritte durchgeführt werden:
• Solubilisieren der aggregierten Chemokinproteine in den Einschlußkörpern;
• Unterziehen der solubilisierten Chemokinproteine einer Umkehrphasenchromatographie;
• Unterziehen des erhaltenen Produkts einem Renaturierungs-/Rückfaltungsschritt;
• Unterziehen des erhaltenen Produkts einem Chromatographieschritt, ausgewählt aus Größenausschlußchromatographie, Ionenaustauschchromatographie, Affinitätschromatographie.

3. Verfahren gemäß Anspruch 1 oder 2, **dadurch gekennzeichnet, daß** die folgenden Schritte durchgeführt werden:
• Solubilisieren der aggregierten Chemokinproteine in den Einschlußkörpern;
• Unterziehen des solubilisierten Chemokinproteins einer Umkehrphasenchromatographie;
• Unterziehen des erhaltenen Produkts einem Renaturierungs-/Rückfaltungsschritt
• Unterziehen des erhaltenen Produkts zwei Ionenaustauschchromatographieschritten.

4. Verfahren gemäß irgendeinem der vorhergehenden Ansprüche, wobei nach dem Solubilisierungs- und/oder Rückfaltungsschritt ein Filtrationsschritt durchgeführt wird.

5. Verfahren gemäß irgendeinem der vorhergehenden Ansprüche, wobei die prokaryotischen Zellen Bakterienzellen sind.

6. Verfahren gemäß Anspruch 5, wobei die Bakterienzellen E. coli-Zellen sind.

7. Verfahren gemäß Anspruch 6, wobei das Chemokin die Chemokinmutante der SEQ ID Nr: 1 ist.

## Revendications

1. Procédé de récupération d'une chimiokine exprimée dans des cellules d'un hôte procaryote telles que des corps d'inclusion et sa purification à la suite, **caractérisé en ce qu'**on interpose une étape de chromatographie en phase inverse entre l'étape de solubilisation des protéines agrégées dans les corps d'inclusion/dénaturation, et l'étape de renaturation/repliement.

2. Procédé selon la revendication 1, **caractérisé en ce que** les étapes suivantes sont effectuées :
- le fait de solubiliser les protéines de chimiokine agrégées dans les corps d'inclusion,
- le fait de soumettre les protéines de chimiokine solubilisées à une chromatographie en phase inverse,
- le fait de soumettre le produit obtenu à une étape de renaturation/repliement,
- le fait de soumettre le produit obtenu à une étape chromatographique choisie parmi la chromatographie d'exclusion de taille, la chromatographie échangeuse d'ions, et la chromatographie d'affinité.

3. Procédé selon la revendication 1 ou 2, **caractérisé en ce que** les étapes suivantes sont effectuées :
- le fait de solubiliser les protéines de chimiokine agrégées dans les corps d'inclusion,
- le fait de soumettre les protéines de chimiokine solubilisées à une chromatographie en phase inverse,
- le fait de soumettre le produit obtenu à une étape de renaturation/repliement,
- le fait de soumettre le produit obtenu à deux étapes de chromatographie échangeuse d'ions.

4. Procédé selon l'une quelconque des revendications précédentes, lequel, après l'étape de solubilisation et/ou de repliement, une étape de filtration est effectuée.

5. Procédé selon l'une quelconque des revendications précédentes, dans lequel les cellules procaryotes sont des cellules bactériennes.

6. Procédé selon la revendication 5, dans lequel les cellules bactériennes sont des cellules d'*E*. *Coli.*

7. Procédé selon la revendication 6, dans lequel la chimiokine est la chimiokine mutante de SEQ ID NO :1.


### SEQUENCE LISTING

<110> APPLIED RESEARCH SYSTEMS ARS HOLDING N.V.
<120> PROCESS FOR THE PURIFICATION OF BACTERIALLY EXPRESSED PROTEINS
<130> EP808Z
<160> 2
<170> PatentIn version 3.1
<210> 1
   <211> 67
   <212> PRT
   <213> Escherichia coli
<400> 1
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Ala Ala Asn Ala Gln
           35                  40                  45
   Val Cys Asn Pro Glu Lys Lys Trp Val Arg Glu Tyr Ile Asn Ser Leu
       50                  55                  60
   Glu Met Ser
   65
<210> 2
   <211> 74
   <212> PRT
   <213> Escherichia coli
<220>
   <221> MISC_FEATURE
   <222> (1)..(7)
   <223> Leader sequence
<400> 2